# EUROPÄISCHE PATENTANMELDUNG

(11) **EP 4 792 782 A1**
(43) Veröffentlichungstag der Anmeldung: **19.08.2026**
(21) Anmeldenummer: 26155909.0
(22) Anmeldetag: 03.02.2026
(51) Int. Cl.: A61F 2/18, A61F 2/28

(54) **SCHLÄFENBEINIMPLANTAT SOWIE VERFAHREN ZUR GESTALTUNG UND/ODER HERSTELLUNG EINES SCHLÄFENBEINIMPLANTATS**

(30) Priorität: 14.02.2025 DE 102025105585
(71) Anmelder: Karl Leibinger Asset Management GmbH & Co. KG, 78570 Mühlheim an der Donau (DE)
(72) Erfinder: Aksu, Adem, 78054 VS-Schwenningen (DE); Reinauer, Frank, 78576 Emmingen-Liptingen (DE)
(74) Vertreter: Schwamberger, Martin

(57) **Zusammenfassung**

Die Erfindung betrifft ein Schläfenbeinimplantat (SI), welches für eine zumindest teilweise Wiederherstellung einer hinteren Wand eines äußeren Gehörganges vorgesehen ist und einen ersten Abschnitt (A1) umfasst, welcher röhren- oder röhrensegmentförmig gestaltet und dazu ausgebildet ist, zumindest einen Wandabschnitt der hinteren Wand durch Überdecken einer Fehlstelle eines Schläfenbeins zu bilden und hierdurch den Gehörgang von der Fehlstelle abzuteilen. Der erste Abschnitt (A1) geht in einen quer zu dem ersten Abschnitt (A1) orientierten, zweiten Abschnitt (A2) über, welcher plattenartig gestaltet ist. Dieser zweite Abschnitt (A2) ist dabei für eine Befestigung an einem Warzenteil des Schläfenbeins und das gemeinsame Überdecken der Fehlstelle mit dem ersten Abschnitt (A1) vorgesehen.

## Beschreibung

Die Erfindung betrifft ein Schläfenbeinimplantat, welches für eine zumindest teilweise Wiederherstellung einer hinteren Wand eines äußeren Gehörganges vorgesehen ist und einen ersten Abschnitt umfasst, welcher röhren- oder röhrensegmentförmig gestaltet und dazu ausgebildet ist, zumindest einen Wandabschnitt der hinteren Wand durch Überdecken einer Fehlstelle eines Schläfenbeins zu bilden und hierdurch den Gehörgang von der Fehlstelle abzuteilen. Des Weiteren betrifft die Erfindung ein Verfahren zur Gestaltung und/oder Herstellung eines Schläfenbeinimplantats.

Bei chronischen Erkrankungen im Bereich des Mittelohres kann in bestimmten Fällen eine operative Behandlung in Form einer radikalen Mastoidektomie erforderlich sein, bei welcher bei einem Schläfenbein (Os temporale) ein Ausräumen eines Warzenteils (Pars mastoidea) unter zumeist teilweiser Entfernung der hinteren Gehörgangswand vorgenommen wird, um eine gemeinsame Höhle aus einem Kuppelraum des Mittelohres, dem Warzenteil und dem Gehörgang zu bilden. Diese Höhle wird dabei häufig auch als Mastoidhöhle oder Radikalhöhle bezeichnet. So kann eine radikale Mastoidektomie im Falle eines ausgeprägten Cholesteatoms notwendig sein, bei welchem es sich um eine chronische Knocheneiterung des Mittelohres handelt. Ziel der radikalen Mastoidektomie ist eine vollständige Entfernung einer Matrix des Cholesteatoms. Neben einer Rekonstruktion der hinteren Gehörgangswand mittels Knorpel(haut)stückchen (teilweise als canal wall up-Technik bezeichnet), sind auch Implantate bekannt, welche für eine Wiederherstellung der hinteren Wand des Gehörganges verwendet werden können.

So offenbart die DE 31 17 025 A1 ein Implantat, welches zur partiellen oder vollständigen Rekonstruktion einer hinteren Gehörgangswand vorgesehen ist, die aufgrund pathologischer Gegebenheiten teilweise oder vollständig zu entfernen und durch das Implantat zu ersetzen ist. Das Implantat umfasst einen röhren- oder röhrensegmentförmigen Körper, welcher aufgrund seiner Form die anatomischen Verhältnisse der hinteren Gehörgangswand abbilden soll.

Ausgehend vom vorstehend beschriebenen Stand der Technik ist es nun die Aufgabe der vorliegenden Erfindung ein Schläfenbeinimplantat zu schaffen, mittels welchem eine möglichst nachbehandlungsarme Wiederherstellung einer hinteren Wand eines Gehörganges möglich sein soll.

Diese Aufgabe wird aus vorrichtungstechnischer Sicht ausgehend vom Oberbegriff des nebengeordneten Anspruchs 1 in Verbindung mit dessen kennzeichnenden Merkmalen gelöst. Aus verfahrenstechnischer Sicht erfolgt eine Lösung der Aufgabe ausgehend vom Oberbegriff des nebengeordneten Anspruchs 11 in Verbindung mit dessen kennzeichnenden Merkmalen. Die hierauf jeweils folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß dem nebengeordneten Anspruch 1 umfasst ein Schläfenbeinimplantat, welches für eine zumindest teilweise Wiederherstellung einer hinteren Wand eines äußeren Gehörganges vorgesehen ist, einen ersten Abschnitt. Dieser erste Abschnitt ist dabei röhren- oder röhrensegmentförmig gestaltet und dazu ausgebildet, zumindest einen Wandabschnitt der hinteren Wand durch Überdecken einer Fehlstelle eines Schläfenbeins zu bilden und hierdurch den Gehörgang von der Fehlstelle abzuteilen.

Das erfindungsgemäße Implantat ist also für die Anwendung bei einem Schläfenbein (Os temporale) konzipiert und dient bei dieser Anwendung der zumindest teilweisen Wiederherstellung einer hinteren Wand des äußeren Gehörganges. Bevorzugt kommt das Schläfenbeinimplantat bei einer operativ gebildeten Mastoidhöhle zur Anwendung, welche häufig auch als Radikalhöhle bezeichnet wird und die durch eine radikale Mastoidektomie gebildet worden ist.

Erfindungsgemäß ist das Schläfenbeinimplantat mit einem ersten Abschnitt versehen, der dazu eingerichtet ist, eine Fehlstelle des Schläfenbeins zu überdecken und aufgrund dieses Überdeckens den wiederherzustellenden, äußeren Gehörgang gegenüber der Fehlstelle abzutrennen. Dabei bildet der erste Abschnitt durch das Abteilen einen Teil der hinteren, knöchernen Gehörgangswand des äußeren Gehörganges (Meatus acusticus externus osseus). Insofern nimmt der erste Abschnitt bei Anwendung des Schläfenbeinimplantats eine Abgrenzung des äußeren Gehörganges von der schläfenbeinseitig gebildeten Fehlstelle vor. Insbesondere wird über den ersten Abschnitt des Schläfenbeinimplantats bei Anwendung eine Fehlstelle in einem Paukenteil (Pars tympanica) des Schläfenbeins überdeckt und diese Paukenteilfehlstelle durch den ersten Abschnitt von dem Gehörgang abgeteilt.

Der erste Abschnitt des Schläfenbeinimplantats ist röhrenförmig oder röhrensegmentförmig gestaltet, worunter im Sinne der Erfindung zu verstehen ist, dass eine Form des ersten Abschnitts ähnlich einer Röhre oder ähnlich eines Segments ausgeführt ist, welches durch Unterteilung einer Röhre entlang ihrer Längsrichtung gebildet ist. Dabei ist diese Form des ersten Abschnitts gewählt, um die Abgrenzung des äußeren Gehörganges gegenüber der Fehlstelle des Schläfenbeins zu verwirklichen und zudem zumindest einen Wandabschnitt der hinteren Wand des äußeren Gehörganges zu bilden. Konkret kann die Röhrenform bzw. Röhrensegmentform des ersten Abschnitts dabei mit unterschiedlichen Innendurchmessern gestaltet und dabei je nach zu rekonstruierendem Verlauf des äußeren Gehörganges auch trichter- oder trichtersegmentförmig ausgeführt sein.

Vorzugsweise weist das Schläfenbeinimplantat eine Innenseite auf, auf welcher das Schläfenbeinimplantat seitens des Schläfenbeins aufzulegen ist und die somit auch als Knochenseite bezeichnet werden kann. Der Innenseite abgewandt liegt hierbei eine Außenseite des Schläfenbeinimplantats, auf welcher Außenseite dann der über den ersten Abschnitt abzuteilende Gehörgang liegt. Insofern kann die Außenseite im Bereich des ersten Abschnitts auch als Gehörgangsseite bezeichnet sein.

Vorzugsweise bildet der erste Abschnitt in seinem Bereich eine geschlossene Trennwand des Schläfenbeinimplantats, d.h. dass an dem ersten Abschnitt keine Durchgänge zwischen der Außenseite und der Innenseite des Schläfenbeinimplantats ausgestaltet sind. Hierdurch kann bei der Abteilung des Gehörgangs von der Fehlstelle über den ersten Abschnitt verhindert werden, dass Körperflüssigkeiten von Seiten der Fehlstelle her durch den ersten Abschnitt hindurch in den äußeren Gehörgang gelangen können. Denn dies würde die Gefahr rezidivierender Entzündungen erhöhen.

Entsprechend dem nebengeordneten Anspruch 1 umfasst die Erfindung nun die technische Lehre, dass der erste Abschnitt in einen quer zu dem ersten Abschnitt orientierten, zweiten Abschnitt übergeht, welcher plattenartig gestaltet ist. Dabei ist der zweite Abschnitt für eine Befestigung an einem Warzenteil des Schläfenbeins und das gemeinsame Überdecken der Fehlstelle mit dem ersten Abschnitt vorgesehen. Mit anderen Worten schließt also an dem ersten Abschnitt ein zweiter Abschnitt des Schläfenbeinimplantats an, wobei dieser zweite Abschnitt quer zu dem ersten Abschnitt verlaufend orientiert und plattenähnlich ausgeführt ist. Bei dem Schläfenbeinimplantat ist der zweite Abschnitt dazu eingerichtet, an einem Warzenteil (Pars mastoidea) des Schläfenbeins befestigt zu werden und gemeinsam mit dem ersten Abschnitt die Fehlstelle des Schläfenbeins abzudecken.

Die erfindungsgemäße Ausgestaltung hat dabei den Vorteil, dass mittels des röhren- oder röhrensegmentförmig gestalteten, ersten Abschnitts und des plattenartig gestalteten, zweiten Abschnitts bei einem Schläfenbein eine zuverlässige Überdeckung einer Fehlstelle verwirklicht werden kann, wie sie insbesondere bei Durchführung einer radikalen Mastoidektomie entsteht. Denn während über den ersten Abschnitt insbesondere eine Fehlstelle im Bereich eines Paukenteils des Schläfenbeins überdeckt werden kann, kann mithilfe des zweiten Abschnitts bevorzugt eine Überdeckung einer Fehlstelle im Bereich des Warzenteils des Schläfenbeins realisiert werden. Darüber hinaus kann an dem zweiten Abschnitt eine zuverlässige Befestigung des Schläfenbeinimplantats am Schläfenbein dargestellt werden, nämlich an dem noch verbleibenden Abschnitt des Warzenteils des Schläfenbeins. Ferner lässt sich über den ersten Abschnitt die Abteilung des Gehörgangs von der Fehlstelle realisieren und dabei die hintere, knöcherne Wand des äußeren Gehörgangs rekonstruieren. Besonders vorteilhaft ist es hierbei, wenn der erste Abschnitt eine geschlossene Trennwand bildet, da hierdurch ein Übergang von Körperflüssigkeiten von der Fehlstelle in den Gehörgang zumindest behindert, bevorzugt gänzlich unterbunden wird. Insgesamt lässt sich also ein Schläfenbeinimplantat verwirklichen, welches für ein Verschließen einer operativ gebildeten Mastoid- oder Radikalhöhle geeignet und dabei aufgrund der Abteilung des Gehörganges von der Fehlstelle nachbehandlungsarm ist. Insbesondere kann hierdurch nach Behandlung eines Cholesteatoms die Gefahr rezidivierender Entzündungen reduziert werden.

Der zweite Abschnitt ist plattenartig gestaltet, worunter im Sinne der Erfindung bevorzugt zu verstehen ist, dass der zweite Abschnitt ähnlich einer Platte eine im Wesentlichen flächige Gestalt aufweist, indem sich der zweite Abschnitt in einem Koordinatensystem hauptsächlich entlang zweier Koordinatenachsen erstreckt, jedoch eine oder mehrere Krümmungen aufweisen kann, um der Anatomie zu folgen. Dabei ist der zweite Abschnitt auf der Innenseite des Schläfenbeinimplantats für eine Befestigung an dem Warzenteil des Schläfenbeins vorgesehen.

Neben der Befestigung an dem Warzenteil ist der zweite Abschnitt des Schläfenbeinimplantats zudem dafür vorgesehen, bei Verwendung des Schläfenbeinimplantats gemeinsam mit dem ersten Abschnitt die Fehlstelle des Schläfenbeins zu überdecken. Während der erste Abschnitt dabei insbesondere dem Überdecken der Fehlstelle im Bereich des Paukenteils des Schläfenbeins dient, ist der zweite Abschnitt bevorzugt für das Überdecken der Fehlstelle im Bereich des Warzenteils des Schläfenbeins vorgesehen. Ganz besonders bevorzugt sind der erste Abschnitt und der zweite Abschnitt des Schläfenbeinimplantats dazu ausgestaltet, gemeinsam die Fehlstelle des Schläfenbeins vollständig zu überdecken.

Vorzugsweise sind der erste Abschnitt und der zweite Abschnitt des Schläfenbeinimplantats einteilig ausgestaltet, wodurch das Schläfenbeinimplantat einstückig verwirklicht wird. Ganz besonders bevorzugt sind der erste Abschnitt und der zweite Abschnitt dabei in einem additiven Herstellungsverfahren ausgestaltet worden, insbesondere im Rahmen eines 3D-Druckverfahrens. Alternativ, bevorzugt aber ergänzend dazu bestehen der erste Abschnitt und der zweite Abschnitt ganz oder abschnittweise aus einem Metall, wie beispielsweise Titan, Edelstahl, Magnesium oder Molybdän, oder aus einer Metalllegierung, beispielsweise einer Titanlegierung, oder aus einem Kunststoff, insbesondere einem Polymer, beispielsweise aus Polyetheretherketon (PEEK), Polyethylen (PE), Polycaprolacton (PCL), oder aus einer Keramik, aus Beta-Trikalziumphosphat (β-TCP), aus Zirconia, aus Hydroxylapatit (HA) oder auch Kombinationen der vorstehend genannten Werkstoffe. Hierbei können zudem nicht poröse Oberflächen der beiden Abschnitte oder poröse Oberflächen der beiden Abschnitte realisiert oder Teilabschnitte der beiden Abschnitte porös und Teilabschnitte nicht porös gestaltet sein.

Gemäß einer Ausführungsform der Erfindung weist der zweite Abschnitt einen Befestigungsteil und einen Abdeckteil auf. Der Befestigungsteil bildet auf einer Innenseite, auf welcher das Schläfenbeinimplantat seitens des Schläfenbeins aufzulegen ist, eine Auflagefläche, an der die Auflage an dem Warzenteil für die Befestigung an dem Warzenteil vorzunehmen ist. Der Abdeckteil ist für das Überdecken der Fehlstelle vorgesehen. Dadurch kann der zweite Abschnitt auf zuverlässige Weise zum einen zur Befestigung seitens des Schläfenbeins sowie zum anderen zur Überdeckung der Fehlstelle gestaltet werden. Bevorzugt umgibt der Befestigungsteil den Abdeckteil peripher abschnittsweise, so dass an dem Befestigungsteil eine Befestigung des Schläfenbeinimplantats an dem Warzenteil umgebend zu der Fehlstelle verwirklicht werden kann.

In Weiterbildung der vorgenannten Ausführungsform können in den Befestigungsteil des zweiten Abschnitts mehreren Aufnahmeöffnungen eingebracht werden, welche von der Innenseite zu einer der Innenseite abgewandt liegenden Außenseite des Schläfenbeinimplantats verlaufen und der Hindurchführung von Befestigungsmitteln dienen. In vorteilhafter Weise kann an diesen Aufnahmeöffnungen die Befestigung des Schläfenbeinimplantats seitens des Schläfenbeins verwirklicht werden. Bevorzugt sind die Aufnahmeöffnungen als kreisrunde Öffnungen gestaltet, welche als Bohrungen in den Befestigungsteil des zweiten Abschnitts abrasiv oder spannend eingebracht sein können. Wurde der zweite Abschnitt im Rahmen eines additiven Herstellungsverfahrens hergestellt, so können die Aufnahmeöffnungen aber auch im Rahmen der additiven Herstellung definiert worden sein. Als Befestigungsmittel können dabei Knochenschrauben zum Verschrauben des Schläfenbeinimplantats mit dem Schläfenbein verwendet oder auch ein Faden zum Vernähen mit dem Schläfenbein verwendet werden.

Alternativ oder ergänzend dazu sind in den Abdeckteil Durchgänge eingebracht, welche von der Innenseite zu einer der Innenseite abgewandt liegenden Außenseite des Schläfenbeinimplantats verlaufen. Diese Durchgänge können dabei als Fadenlöcher zur Befestigung von Haut an dem Abdeckteil des zweiten Abschnitts verwendet werden, insbesondere dann, wenn der zweite Teil aus einem Material besteht, an welchem ein Anwachsen von Gewebe erschwert oder unmöglich ist.

Entsprechend einer Ausgestaltungsmöglichkeit der Erfindung bildet der erste Abschnitt auf einer Innenseite, auf welcher das Schläfenbeinimplantat seitens des Schläfenbeins aufzulegen ist, eine Dichtfläche, welche für eine Anlage an dem Schläfenbein vorgesehen ist. In vorteilhafter Weise kann hierdurch eine Abdichtung des Gehörgangs gegenüber der Fehlstelle des Schläfenbeins realisiert werden. Besonders vorteilhaft ist es, wenn zudem an der Dichtfläche eine Beschichtung aufgebracht ist, da hierdurch eine besonders zuverlässige Abdichtung realisierbar ist. Bei dieser Beschichtung kann es sich dabei um ein flexibles Material, wie beispielsweise Silikon, poröses Polyetheretherketon (PEEK), poröses Polyethylen (PE), Polycaprolacton (PCL) oder Ähnliches handeln. Zudem könnte die Beschichtung ggf. resorbierbar ausgeführt sein.

In Weiterbildung der vorgenannten Ausgestaltungsmöglichkeit kann die Dichtfläche zumindest abschnittsweise an einer Peripherie des ersten Teils ausgestaltet und dafür vorgesehen sein, den Gehörgang durch Anlage an dem Schläfenbein gegenüber der Fehlstelle abzudichten. Besonders bevorzugt ist an der Dichtfläche im Bereich des ersten Teils eine fortlaufende Anlage an dem Schläfenbein realisiert, um bei der Abteilung des Gehörgangs von der Fehlstelle eine möglichst geschlossene Abdichtung zu verwirklichen.

Es ist eine weitere mögliche Ausführungsform der Erfindung, dass der erste Abschnitt und der zweite Abschnitt patientenspezifisch geformt sind. Dies hat den Vorteil, dass der erste Abschnitt und der zweite Abschnitt und damit auch das Schläfenbeinimplantat insgesamt an das Schläfenbein des jeweiligen Patienten angepasst sind. Bevorzugt sind der erste Abschnitt und der zweite Abschnitt Knochensegmenten nachempfunden, welche patientenseitig bei dem Schläfenbein zu entfernen sind. Insbesondere sind dazu im Vorfeld Konturen des Schläfenbeins im Rahmen eines medizinischen, bildgebenden Verfahrens erfasst worden, wie beispielsweise mittels CT.

Gemäß dem nebengeordneten Anspruch 11 betrifft die Erfindung zudem ein Verfahren zur Gestaltung und/oder Herstellung eines Schläfenbeinimplantats, welches für eine zumindest teilweise Wiederherstellung einer hinteren Wand eines äußeren Gehörganges vorgesehen ist. Dabei wird bei dem Schläfenbeinimplantat ein röhren- oder röhrensegmentförmiger, erster Abschnitt dazu ausgebildet, zumindest einen Teil der hinteren Wand durch Überdecken einer Fehlstelle eines Schläfenbeins zu bilden und hierdurch den Gehörgang von der Fehlstelle abzuteilen. Zudem wird bei dem Schläfenbeinimplantat neben dem ersten Abschnitt ein zweiter Abschnitt gestaltet, welcher hierbei plattenartig und quer zu dem ersten Abschnitt orientiert ausgebildet wird, wobei der zweite Abschnitt für eine Befestigung an einem Warzenteil des Schläfenbeins und das gemeinsame Überdecken der Fehlstelle mit dem ersten Abschnitt ausgeführt wird.

Im Rahmen des erfindungsgemäßen Verfahrens wird also eine Gestaltung oder eine Herstellung oder sowohl eine Gestaltung als auch eine Herstellung eines Schläfenbeinimplantats durchgeführt. Dabei ist unter einer "Gestaltung" insbesondere eine Planung bzw. Konstruktion des Schläfenbeinimplantats zu verstehen, also eine Erstellung eines Entwurfs bzw. einer Vorlage für eine Herstellung des Schläfenbeinimplantats. Mit "Herstellung" ist eine Fertigung des Schläfenbeinimplantats gemeint, also dessen physische Ausgestaltung. Insbesondere findet im Rahmen des erfindungsgemäßen Verfahrens sowohl die Gestaltung als auch die Herstellung eines Schläfenbeinimplantats statt. Ganz besonders bevorzugt findet die Herstellung in einem additiven Herstellungsverfahren statt, insbesondere mittels eines 3D-Druckverfahrens.

In vorteilhafter Weise kann somit ein Schläfenbeinimplantat gestaltet und/oder hergestellt werden, mittels welchem eine zuverlässige Überdeckung einer Fehlstelle darstellbar ist, wie sie insbesondere bei einer radikalen Mastoidektomie entsteht. Des Weiteren kann das gestaltete bzw. hergestellte Schläfenbeinimplantat dann dazu verwendet werden, über den ersten Abschnitt eine Abteilung des Gehörgangs von der Fehlstelle zu realisieren und damit eine hintere, knöcherne Wand des Gehörgangs (Meatus acusticus externus osseus) zu rekonstruieren. Insgesamt wird hierdurch ein Schläfenbeinimplantat verwirklicht, welches für ein Verschließen einer operativ gebildeten Mastoid- oder Radikalhöhle geeignet und dabei aufgrund der Abteilung des Gehörganges von der Fehlstelle nachbehandlungsarm ist.

Bevorzugt wird das Schläfenbeinimplantat bei der Gestaltung und/oder bei der Erstellung zudem entsprechend wenigstens einer der vorstehend beschriebenen Varianten eines erfindungsgemäßen Schläfenbeinimplantats ausgestaltet.

In Weiterbildung des erfindungsgemäßen Verfahrens können der erste Abschnitt und der zweite Abschnitt bei der Gestaltung Knochensegmenten nachempfunden werden, welche patientenseitig bei dem Schläfenbein zu entfernen sind. Hierdurch kann eine patientenspezifische Anpassung des Schläfenbeinimplantats realisiert werden. Alternativ, bevorzugt aber ergänzend dazu wird die Gestaltung des ersten Abschnitts und des zweiten Abschnitts anhand von im Vorfeld bestimmten Konturen des Schläfenbeins vorgenommen, wobei diese Bestimmung dabei insbesondere im Rahmen eines medizinischen, bildgebenden Verfahrens, wie beispielsweise einem CT, stattgefunden hat.

Es ist eine weitere Ausgestaltungsmöglichkeit des erfindungsgemäßen Verfahrens, dass gemeinsam mit dem Schläfenbeinimplantat zudem eine Operationsschablone geplant und/oder hergestellt wird. In vorteilhafter Weise kann diese Operationsschablone dann zur Durchführung der radikalen Mastoidektomie verwendet werden, wobei hierbei eine passgenaue Entfernung von Segmenten des Schläfenbeins darstellbar ist.

Eine vorteilhafte Ausführungsform der Erfindung, die nachfolgend erläutert wird, ist in den Zeichnungen dargestellt. Es zeigen:
- Fig. 1: eine perspektivische Darstellung eines Schläfenbeinimplantats entsprechend einer bevorzugten Ausführungsform;
- Fig. 2: eine perspektivische Ansicht eines Teils eines Schädels im Bereich eines Schläfenbeins mit einem markierten, zu entfernenden Bereich des Schläfenbeins;
- Fig. 3: eine weitere perspektivische Darstellung des Bereichs des Schädels aus Fig. 2, mit einer aufgesetzten Operationsschablone;
- Fig. 4: eine weitere perspektivische Darstellung des Bereichs des Schädels aus Fig. 2 nach einem Entfernen des Bereichs des Schläfenbeins; und
- Fig. 5 bis 7: unterschiedliche Ansichten des Schädels nach einem Einsetzen des Schläfenbeinimplantats.

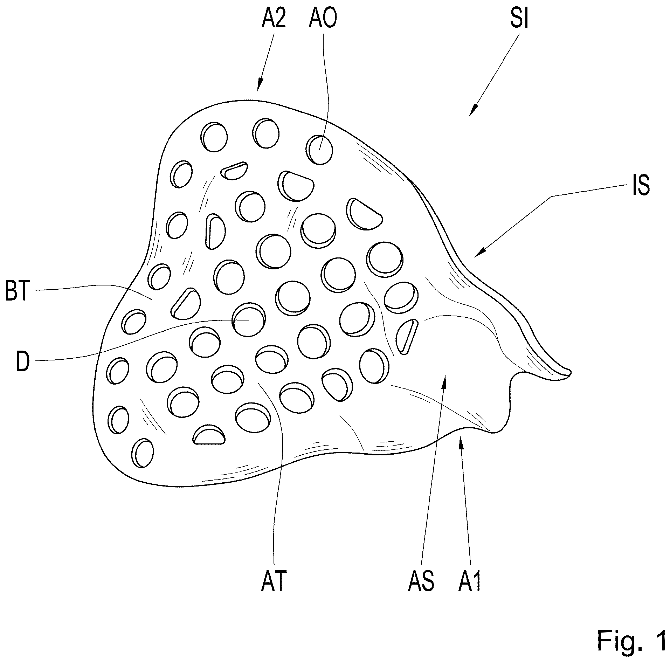

Aus Fig. 1 geht eine perspektivische Darstellung eines Schläfenbeinimplantats SI hervor, welches entsprechend einer bevorzugten Ausführungsform der Erfindung gestaltet ist. Dieses Schläfenbeinimplantat SI ist einteilig im Rahmen eines additiven Herstellungsverfahrens hergestellt worden, wobei diese Herstellung mittels eines 3D-Druckverfahrens stattgefunden hat.

Vorliegend besteht das Schläfenbeinimplantat SI aus Titan, wobei alternativ dazu aber auch ein anderer metallischer Werkstoff, wie Edelstahl oder Magnesium, eine Metalllegierung, beispielsweise einer Titanlegierung, ein Kunststoff, insbesondere ein Polymer, beispielsweise Polyetheretherketon (PEEK), Polyethylen (PE), Polycaprolacton (PCL), eine Keramik, Beta-Tricalciumphosphat (β-TCP), Zirconia oder Hydroxylapatit (HA) sowie auch eine Kombination dieser Werkstoffe verwendet worden sein könnte.

Das Schläfenbeinimplantat SI setzt sich aus einem ersten Abschnitt A1 und einem zweiten Abschnitt A2 zusammen. Der erste Abschnitt A1 ist dabei röhrensegmentförmig gestaltet, indem der erste Abschnitt A1 mit einer Wölbung ausgeführt ist, die zu einer Außenseite AS des Schläfenbeinimplantats SI hin gerichtet ist. Der erste Abschnitt A1 geht in den zweiten Abschnitt A2 über, welcher hierbei quer zu dem ersten Abschnitt A1 orientiert und plattenförmig gestaltet ist.

Der zweite Abschnitt A2 verfügt über einen Abdeckteil AT und einen Befestigungsteil BT, welcher den Abdeckteil AT auf einer Außenperipherie umgibt. In den Befestigungsteil sind dabei mehrere Aufnahmeöffnungen AO als kreisrunde Öffnungen ausgestaltet, die hierbei jeweils von der Außenseite AS zu einer der Außenseite AS bei dem Schläfenbeinimplantat SI abgewandt liegenden Innenseite IS verlaufen und den Befestigungsteil BT vollständig durchdringen.

Wie in Fig. 1 zu erkennen ist, ist der Abdeckteil AT des zweiten Abschnitts A2 mit Durchgängen D versehen, die den Abdeckteil AT des zweiten Abschnitts A2 vollständig von der Außenseite AS zu der Innenseite IS hin durchdringen. Hingegen ist der erste Abschnitt A1 bei dem Schläfenbeinimplantat SI als massive Wand ausgeführt und weist keine Durchgänge oder Öffnungen zwischen der Außenseite AS und der Innenseite IS auf.

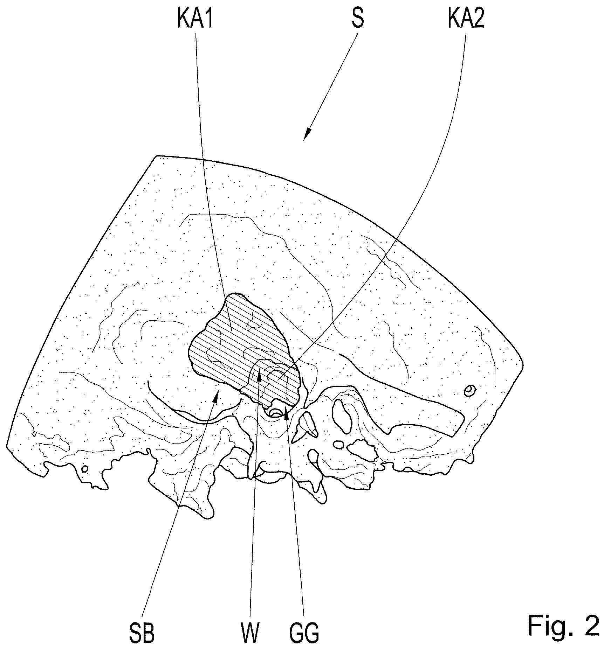

Das in Fig. 1 dargestellte Schläfenbeinimplantat SI ist für die konkrete, patientenspezifische Anwendung im Bereich eines Schläfenbeins SB eines Schädels S gestaltet und hergestellt worden. Dazu wurde die Gestalt dieses Schädels S im Vorfeld mittels eines medizinischen, bildgebenden Verfahrens, insbesondere mittels CT, unter Berücksichtigung eines in den Fig. 2 bis 7 jeweils teilweise dargestellten 3D-Modells des Schädels S ermittelt. Hierbei ist das Schläfenbeinimplantat SI dadurch patientenspezifisch ausgestaltet, indem die beiden Abschnitte A1 und A2 des Schläfenbeinimplantats SI in Anlehnung an Knochenabschnitte KA1 und KA2 gestaltet sind, welche bei dem Schläfenbein SB des Schädels S als im Rahmen einer radikalen Mastoidektomie zu entfernende Bereiche deklariert worden und in Fig. 2 schraffiert angedeutet sind. Die radikale Mastoidektomie ist dabei insbesondere dazu durchzuführen, um ein Cholesteatom im Bereich eines äußeren Gehörgangs GG vollständig entfernen zu können.

Vorliegend handelt es sich bei dem zu entfernenden Knochenabschnitt KA1 um einen Abschnitt eines Warzenteils (Pars mastoidea) des Schläfenbeins SB, während der zu entfernende Knochenabschnitt KA2 ein Abschnitt eines Paukenteils (Pars tympanica) des Schläfenbeins SB ist und hierbei einen Teil der hinteren, knöchernen Wand W des äußeren Gehörganges GG bildet.

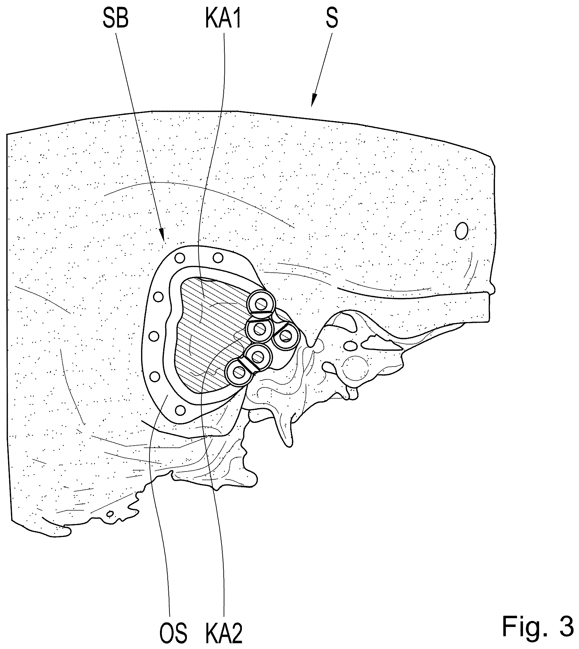

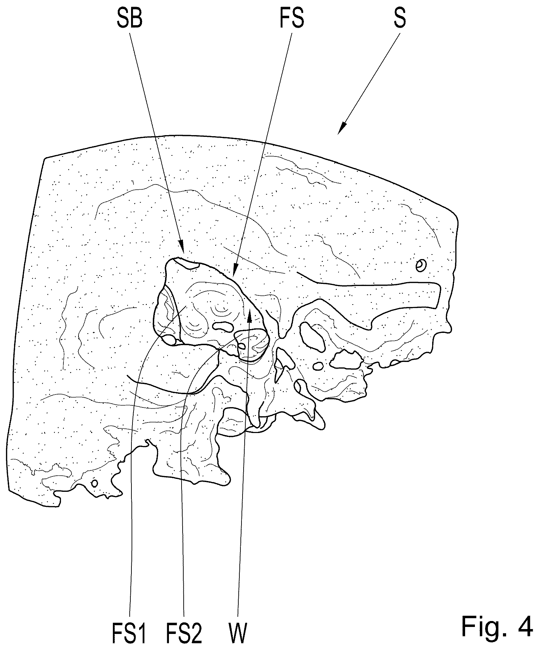

Fig. 3 zeigt den Schädel S mit einer aufgesetzten Operationsschablone OS, welche gemeinsam mit dem Schläfenbeinimplantat SI auf Basis der erfassten Gestalt des Schädels S patientenspezifisch gestaltet worden ist. Dabei ist diese Operationsschablone OS, wie anhand von Fig. 3 zu erkennen ist, für eine Befestigung am Schläfenbein SB konzipiert und definiert als Säge- und Bohrschablone die zu entfernenden Knochenabschnitte KA1 und KA2. Ferner ist der Schädel S in Fig. 4 nach einem Entfernen der Knochenabschnitte KA1 und KA2 gezeigt, wodurch sich bei dem Schläfenbein SB eine Fehlstelle FS gebildet hat. Dadurch, dass der Knochenabschnitte KA1 einen Abschnitt des Warzenteils und der Knochenabschnitt KA2 einen Abschnitt des Paukenteils bildet, weist die Fehlstelle FS einen Fehlstellenteil FS1 im Bereich des Warzenteils und einen Fehlstellenteil FS2 im Bereich des Paukenteils auf, wobei aufgrund Letzterem ein Abschnitt der Wand W entfernt worden ist.

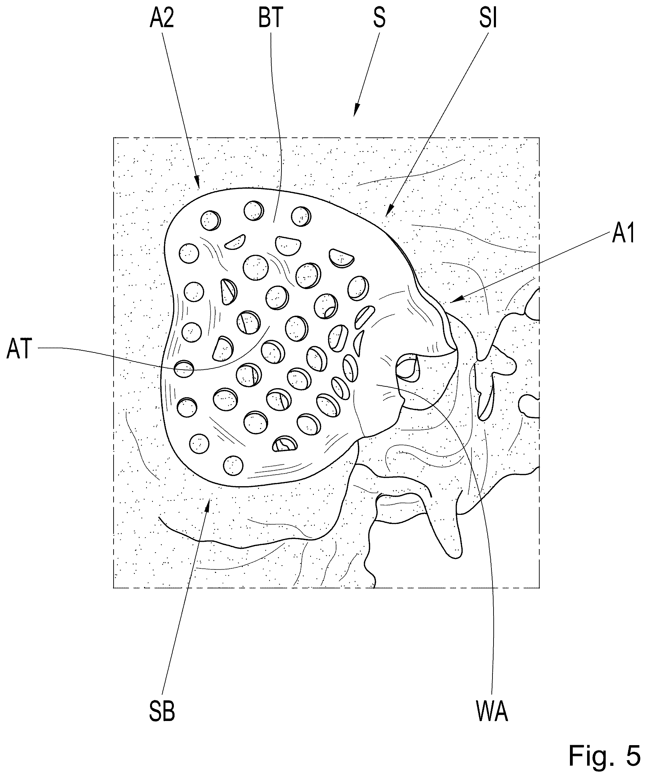

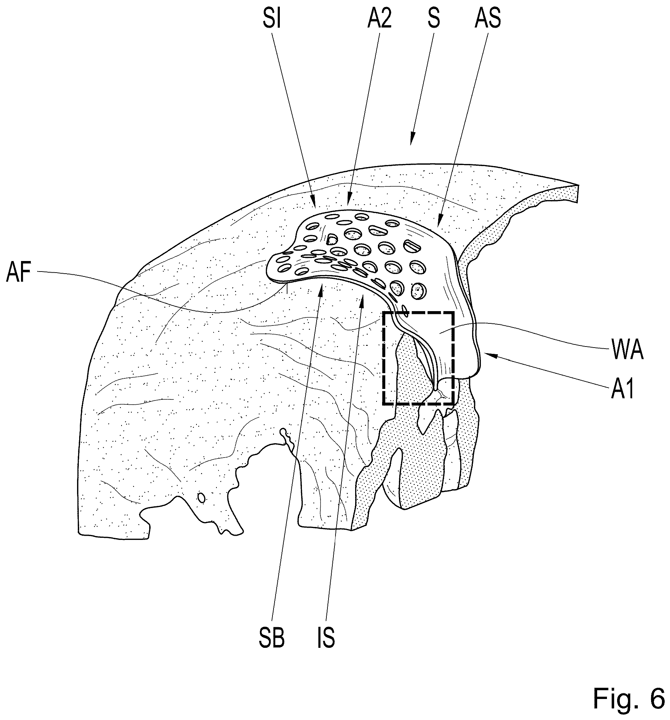

In Fig. 5 bis Fig. 7 ist der Schädel S nun jeweils mit dem am Schläfenbein SB angeordneten Schläfenbeinimplantat SI gezeigt, welches dazu an dem Befestigungsteil BT des zweiten Abschnitts A2 auf den Warzenteil des Schläfenbeins SB aufgelegt worden ist. Wie dabei insbesondere anhand von Fig. 6 zu erkennen ist, ist diese Auflage dabei auf der Innenseite IS des Schläfenbeinimplantats SI an einer Auflagefläche AF vorgenommen, mit welcher der Befestigungsteil BT auf der Innenseite IS ausgestattet ist. An den Aufnahmeöffnungen AO des Befestigungsteils BT kann dabei eine Befestigung des Schläfenbeinimplantats SI am Schläfenbein SB vorgenommen werden, wozu entsprechende, in den Figuren nicht dargestellte Befestigungsmittel durch die Aufnahmeöffnungen AO hindurchzuführen sind. Im Rahmen der Erfindung kann es sich bei diesen Befestigungsmitteln um Knochenschrauben zum Verschrauben oder auch um einen Faden zum Vernähen mit dem Schläfenbein SB handeln. Zudem können die Durchgänge D des Abdeckteils AT als Fadenlöcher für ein Vernähen des Schläfenbeinimplantats SI mit Gewebe, insbesondere Haut, verwendet werden.

Während über den Abdeckteil AT des zweiten Abschnitts A2 der Fehlstellenteil FS1 überdeckt wird, überdeckt der erste Abschnitt A1 den Fehlstellenteil FS2 und bildet hierbei zudem einen Wandabschnitt WA der hinteren Wand W. Hierdurch teilt der erste Abschnitt A1 den äußeren Gehörgangs GG von der Fehlstelle FS ab. Über die beiden Abschnitte A1 und A2 ist zudem eine vollständige Überdeckung der Fehlstelle FS verwirklicht.

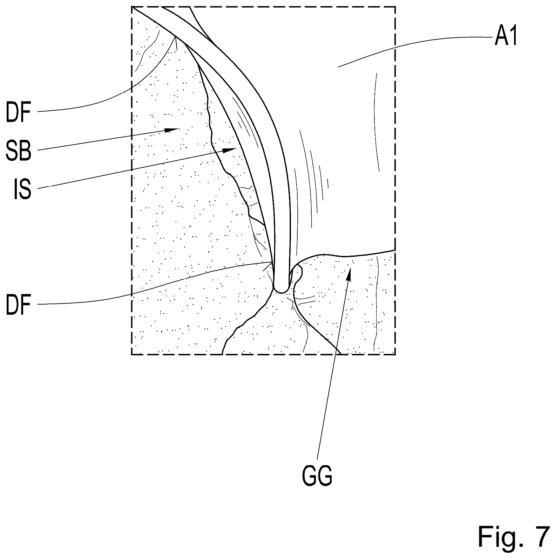

Wie zudem anhand einer Detailansicht in Fig. 7 erkennbar ist, bildet der erste Abschnitt A1 auf der Innenseite IS an einer Peripherie eine Dichtfläche DF, an welcher der erste Abschnitt A1 umlaufend an einer äußeren Umrandung des Fehlstellenteils FS2 an dem Schläfenbein SB anliegt und hierdurch den äußeren Gehörgangs GG gegenüber der Fehlstelle FS abdichtet. Durch diese umlaufende Anlage und der Ausführung des ersten Abschnitts A1 als massive Wand wird mithilfe des Schläfenbeinimplantats SI ein Eindringen von Körperflüssigkeiten über die Fehlstelle FS in den äußeren Gehörgangs GG unterbunden. Um die Abdichtung dabei weiter zu verbessern, kann der erste Abschnitt A1 an der Dichtfläche DF zudem mit einer Beschichtung versehen sein.

Mittels der erfindungsgemäßen Ausgestaltung kann somit ein Schläfenbeinimplantat geschaffen werden, mittels welchem eine nachbehandlungsarme Wiederherstellung einer hinteren Wand eines äußeren Gehörganges möglich ist.

### Bezugszeichenliste

- SI: Schläfenbeinimplantat
- A1: erster Abschnitt
- A2: zweiter Abschnitt
- AS: Außenseite
- AT: Abdeckteil
- BT: Befestigungsteil
- AO: Aufnahmeöffnungen
- IS: Innenseite
- D: Durchgänge
- SB: Schläfenbein
- S: Schädel
- KA1: Knochenabschnitt
- KA2: Knochenabschnitt
- GG: äußerer Gehörgang
- W: Wand
- OS: Operationsschablone
- FS: Fehlstelle
- FS1, FS2: Fehlstellenteile
- AF: Auflagefläche
- WA: Wandabschnitt
- DF: Dichtfläche

## Patentansprüche

1. Schläfenbeinimplantat (SI), welches für eine zumindest teilweise Wiederherstellung einer hinteren Wand (W) eines äußeren Gehörganges (GG) vorgesehen ist und einen ersten Abschnitt (A1) umfasst, welcher röhren- oder röhrensegmentförmig gestaltet und dazu ausgebildet ist, zumindest einen Wandabschnitt (WA) der hinteren Wand (W) durch Überdecken einer Fehlstelle (FS) eines Schläfenbeins (SB) zu bilden und hierdurch den Gehörgang (GG) von der Fehlstelle (FS) abzuteilen, **dadurch gekennzeichnet, dass** der erste Abschnitt (A1) in einen quer zu dem ersten Abschnitt (A1) orientierten, zweiten Abschnitt (A2) übergeht, welcher plattenartig gestaltet ist, und dass der zweite Abschnitt (A2) für eine Befestigung an einem Warzenteil des Schläfenbeins (SB) und das gemeinsame Überdecken der Fehlstelle (FS) mit dem ersten Abschnitt (A1) vorgesehen ist.

2. Schläfenbeinimplantat (SI) nach Anspruch 1, **dadurch gekennzeichnet, dass** der zweite Abschnitt (A2) einen Befestigungsteil (BT) und einen Abdeckteil (AT) aufweist, wobei der Befestigungsteil (BT) auf einer Innenseite (IS), auf welcher das Schläfenbeinimplantat (SI) seitens des Schläfenbeins (SB) aufzulegen ist, eine Auflagefläche (AF) bildet, an der die Auflage an dem Warzenteil für die Befestigung an dem Warzenteil vorzunehmen ist, und wobei der Abdeckteil (AT) für das zumindest abschnittsweise Überdecken der Fehlstelle (FS) vorgesehen ist.

3. Schläfenbeinimplantat (SI) nach Anspruch 2, **dadurch gekennzeichnet, dass** der Befestigungsteil (BT) den Abdeckteil (AT) peripher abschnittsweise umgibt.

4. Schläfenbeinimplantat (SI) nach Anspruch 2 oder 3, **dadurch gekennzeichnet, dass** in den Befestigungsteil (BT) des zweiten Abschnitts (A2) mehreren Aufnahmeöffnungen (AO) eingebracht sind, welche von der Innenseite (IS) zu einer der Innenseite (IS) abgewandt liegenden Außenseite (AS) des Schläfenbeinimplantats (SI) verlaufen und der Hindurchführung von Befestigungsmitteln dienen.

5. Schläfenbeinimplantat (SI) nach wenigstens einem der Ansprüche 2 bis 4, **dadurch gekennzeichnet, dass** in den Abdeckteil (AT) Durchgänge (D) eingebracht sind, welche von der Innenseite (IS) zu einer der Innenseite (IS) abgewandt liegenden Außenseite (AS) des Schläfenbeinimplantats (SI) verlaufen.

6. Schläfenbeinimplantat (SI) nach wenigstens einem der vorhergehenden Ansprüche, **dadurch gekennzeichnet, dass** der erste Abschnitt (A1) auf einer Innenseite (IS), auf welcher das Schläfenbeinimplantat (SI) seitens des Schläfenbeins (SB) aufzulegen ist, eine Dichtfläche (DF) bildet, welche für eine Anlage an dem Schläfenbein (SB) vorgesehen ist.

7. Schläfenbeinimplantat (SI) nach Anspruch 6, **dadurch gekennzeichnet, dass** an der Dichtfläche (DF) eine Beschichtung aufgebracht ist.

8. Schläfenbeinimplantat (SI) nach Anspruch 6 oder 7, **dadurch gekennzeichnet, dass** die Dichtfläche (DF) zumindest abschnittsweise an einer Peripherie des ersten Teils (A1) ausgestaltet und dafür vorgesehen ist, den Gehörgang (GG) durch Anlage an dem Schläfenbein (SB) gegenüber der Fehlstelle (FS) abzudichten.

9. Schläfenbeinimplantat (SI) nach wenigstens einem der vorhergehenden Ansprüche, **dadurch gekennzeichnet, dass** der erste Abschnitt (A1) und der zweite Abschnitt (A2) patientenspezifisch geformt sind.

10. Schläfenbeinimplantat (SI) nach Anspruch 9, **dadurch gekennzeichnet, dass** der erste Abschnitt (A1) und der zweite Abschnitt (A2) Knochenabschnitten (KA1, KA2) nachempfunden sind, welche patientenseitig bei dem Schläfenbein (SB) zu entfernen sind.

11. Verfahren zur Gestaltung und/oder Herstellung eines Schläfenbeinimplantats (SI), welches für eine zumindest teilweise Wiederherstellung einer hinteren Wand (W) eines äußeren Gehörganges (GG) vorgesehen ist, wobei bei dem Schläfenbeinimplantat (SI) ein röhren- oder röhrensegmentförmiger, erster Abschnitt (A1) dazu ausgebildet wird, zumindest einen Teil der hinteren Wand (W) durch Überdecken einer Fehlstelle (FS) eines Schläfenbeins (SB) zu bilden und hierdurch den Gehörgang (GG) von der Fehlstelle (FS) abzuteilen, wobei bei dem Schläfenbeinimplantat (SI) neben dem ersten Abschnitt (A1) ein zweiter Abschnitt (A2) gestaltet wird, welcher hierbei plattenartig und quer zu dem ersten Abschnitt (A1) orientiert ausgebildet wird, und wobei der zweite Abschnitt (A2) für eine Befestigung an einem Warzenteil des Schläfenbeins (SB) und das gemeinsame Überdecken der Fehlstelle (FS) mit dem ersten Abschnitt (A1) ausgeführt wird.

12. Verfahren nach Anspruch 11, **dadurch gekennzeichnet, dass** das Schläfenbeinimplantat (SI) im Weiteren nach wenigstens einem der Ansprüche 2 bis 10 gestaltet wird.

13. Verfahren nach Anspruch 11 oder 12, **dadurch gekennzeichnet, dass** der erste Abschnitt (A1) und/oder der zweite Abschnitt (A2) bei der Gestaltung Knochenabschnitten (KA1, KA2) nachempfunden werden, welche patientenseitig bei dem Schläfenbein (SB) zu entfernen sind.

14. Verfahren nach wenigstens einem der Ansprüche 11 bis 13, **dadurch gekennzeichnet, dass** die Gestaltung des ersten Abschnitts (A1) und des zweiten Abschnitts (A2) anhand von im Vorfeld bestimmten Konturen des Schläfenbeins (SB) vorgenommen wird.

15. Verfahren nach wenigstens einem der Ansprüche 11 bis 14, **dadurch gekennzeichnet, dass** gemeinsam mit dem Schläfenbeinimplantat (SI) zudem eine Operationsschablone (OS) geplant und/oder hergestellt wird.
